# EUROPÄISCHE PATENTANMELDUNG

(11) **EP 2 801 563 A1**
(43) Veröffentlichungstag der Anmeldung: **12.11.2014**
(21) Anmeldenummer: 13166648.9
(22) Anmeldetag: 06.05.2013
(51) Int. Cl.: C07C 37/50

(54) **Decarboxylierung von 6-Methylsalicylsäure**

(71) Anmelder: LANXESS Deutschland GmbH, 50569 Köln (DE)
(72) Erfinder: Schlummer, Björn, 53225 Bonn (DE); Vogl, Nadine, 50733 Köln (DE); Dreisbach, Claus, 42799 Leichlingen (DE)

(57) **Zusammenfassung**

Die vorliegende Erfindung betrifft ein besonders einfaches Verfahren zur Decarboxylierung von 6-Methylsalicylsäure mittels Base-Katalysatoren.

## Beschreibung

Die vorliegende Erfindung betrifft ein besonders einfaches Verfahren zur Decarboxylierung von 6-Methylsalicylsäure (6-MSA).

Aus Berichte der Deutschen Chemischen Gesellschaft, Berlin, Verlag Chemie, Jahrg. 1883, Seite 1963 ist bekannt, 6-Methylsalicylsäure, auch als β-Metahomosalicylsäure bezeichnet, durch Erhitzen mit konzentrierter Salzsäure auf 200°C in Kohlensäure und meta-Kresol zu spalten.

Aus WO 2012/178110 A2 ist die Decarboxylierung von 6-MSA durch Erhitzen mit Metallkatalysatoren bekannt. Explizit werden dort 20 g 6-MSA mit 2,5 g Zinkpulver decarboxyliert und 11,35 g m-Kresol in einer Reinheit von über 99,9 % erhalten.

Ausgehend von diesem Stand der Technik bestand die Aufgabe der vorliegenden Erfindung darin, alternative Katalysatoren für die Decarboxylierung von 6-MSA bereit zu stellen. Für industrielle Anwendungen wäre der Aufwand des Einsatzes von 0,0382 Mol Zink für 0,1315 Mol 6-MSA gemäß Beispiel 7 der WO 2012/178110 A2, um daraus 0,1 Mol m-Kresol zu erhalten, nicht rentabel und zudem mit hohem Aufwand hinsichtlich der Entsorgung des verbrauchten Zink-Katalysators verbunden. Für jeden Einsatz müsste neuer Zink-Katalysator bereit gestellt werden und dieser nach dem Einsatz entsorgt werden, wodurch allenfalls eine Batch-Fahrweise möglich wäre.

Es wurde nun überraschend gefunden, dass als Katalysatoren sowohl alternative Säuren zu Salzsäure (HCl) aber auch Basen in deutlich effektiverer Weise 6-MSA decarboxylieren.

Gegenstand der vorliegenden Erfindung ist ein Verfahren zur Decarboxylierung von 6-MSAI, dadurch gekennzeichnet, dass man als Katalysator wenigstens eine Base der Reihe Alkalimetallhydroxid, Erdalkalimetallhydroxid oder Erdalkalimetalloxid, bevorzugt Base der Reihe CaO, Ca(OH)₂, Ba(OH)₂, Mg(OH)₂, NaOH, LiOH oder KOH, einsetzt.

Erfindungsgemäß bevorzugt entsteht meta-Kresol (m-Kresol).

Zur Klarstellung sei angemerkt, dass vom Rahmen dieser Erfindung alle nachfolgend aufgeführten, allgemeinen oder in Vorzugsbereichen genannten Definitionen und Parameter in beliebigen Kombinationen umfasst sind.

Gegenüber dem Stand der Technik haben die erfindungsgemäß einzusetzenden Katalysatoren den Vorteil, dass m-Kresol in deutlich höherer Ausbeute erhalten wird und ein Eintrag von Schwermetallen ins m-Kresol unterbleibt. Die Anwesenheit von Schwermetallen im m-Kresol kann dessen Einsatz in der Pharmazie oder im Pflanzenschutz erheblich einschränken, wenn nicht das m-Kresol umfangreichen Aufarbeitungsschritten zuvor unterzogen wird.

In einer Ausführungsform betrifft die vorliegende Erfindung ein Verfahren zur Herstellung von m-Kresol indem man ausgehend von 6-MSA wenigstens Base der Reihe Alkalimetallhydroxid, Erdalkalimetalloxid oder Erdalkalimetallhydroxid, bevorzugt wenigstens eine Base der Reihe CaO, Ca(OH)₂, Ba(OH)₂, Mg(OH)₂, NaOH, LiOH oder KOH, einsetzt.

Die vorliegende Erfindung betrifft ferner die Verwendung von wenigstens einer Base der Reihe Alkalimetallhydroxid, Erdalkalimetalloxid oder Erdalkalimetallhydroxid, bevorzugt wenigstens eine Base der Reihe CaO, Ca(OH)₂, Ba(OH)₂, Mg(OH)₂, NaOH, LiOH oder KOH, als Katalysator zur Decarboxylierung von 6-MSA.

In einer Ausführungsform betrifft die vorliegende Erfindung ein Verfahren zur Decarboxylierung von 6-MSA, dadurch gekennzeichnet, dass man als Katalysator wenigstens eine Base der Reihe Alkalimetallhydroxid, Erdalkalimetalloxid oder Erdalkalimetallhydroxid, bevorzugt wenigstens eine Base der Reihe CaO, Ca(OH)₂, Ba(OH)₂, Mg(OH)₂, NaOH, LiOH oder KOH, einsetzt.

In einer Ausführung der vorliegenden Erfindung wird als Katalysator Ca(OH)₂ eingesetzt.

In einer Ausführung der vorliegenden Erfindung wird als Katalysator Ba(OH)₂ eingesetzt.

In einer Ausführung der vorliegenden Erfindung wird als Katalysator Mg(OH)₂ eingesetzt.

In einer Ausführung der vorliegenden Erfindung wird als Katalysator KOH eingesetzt.

In einer Ausführung der vorliegenden Erfindung wird als Katalysator CaO eingesetzt.

In einer Ausführungsform wird das erfindungsgemäße Verfahren lösungsmittelfrei durchgeführt und ist damit dem Verfahren des Standes der Technik in der Durchführung technisch überlegen.

In einer Ausführungsform wird das erfindungsgemäße Verfahren bei einer Temperatur im Bereich von 180°C-200°C, bevorzugt im Bereich von 190°C-200°C durchgeführt. Die experimentellen Untersuchungen zur vorliegenden Erfindung wurden bei 195°C durchgeführt.

In einer Ausführungsform kann 6-MSA auf petrochemischer Basis oder aus mikrobiologischen Syntheseprozessen eingesetzt werden.

In einer Ausführungsform kann das erfindungsgemäße Verfahren in Gegenwart der 0,5-bis 15-fachen Gewichtsmenge Wasser, bezogen auf die einzusetzende Menge 6-MSA durchgeführt werden. Bevorzugt beträgt diese Menge das 0,8- bis 6-fache.

In einer Ausführungsform kann das erfindungsgemäße Verfahren in Gegenwart zusätzlicher Metallkatalysatoren durchgeführt werden. Als Metallkatalysatoren werden bevorzugt Metalle der Reihe Zn, Fe, Cu, Ni, Co, Pd, Mo, Ru oder deren chemische Verbindungen mit Nichtmetallen bzw. Salze der Reihe Oxide, Chloride, Acetate oder deren Komplexe mit CO oder Dibenzylidenaceton eingesetzt. Vorzugsweise werden diese Katalysatoren in Mengen von 10 bis 100 mol-% bezogen auf 1 Mol 6-MSA zugegeben.

Üblicherweise erhitzt man die 6-MSA gegebenenfalls in Gegenwart von Wasser und in Gegenwart von wenigstens einem der erfindungsgemäß einzusetzenden Säure- oder Basen-Katalysatoren, vorzugsweise auf eine Temperatur von 180 bis 200 °C. Wenn die Reaktionstemperatur bei Normaldruck über der Siedetemperatur des Reaktionsgemisches liegt, ist es erforderlich, die Decarboxylierung in einem druckfesten Reaktor, bevorzugt in einem Autoklaven, durchzuführen.

Die Reaktionszeiten können in einem weiten Bereich variiert werden und vorzugsweise im Bereich von 1 bis 60 Stunden liegen. Im Allgemeinen kann bei höheren Reaktionstemperaturen mit kürzeren Reaktionszeiten gearbeitet werden als bei tieferen Reaktionszeiten. Es ist ein wesentliches Merkmal des erfindungsgemäßen Verfahrens, dass man es ohne Zusätze von organischen Lösungsmitteln durchführt.

Das nach der Durchführung der erfindungsgemäßen Decarboxylierung vorliegende Reaktionsmedium wird in einer bevorzugten Ausführungsform direkt destilliert, wodurch man das m-Kresol isoliert. Man erhält so im Allgemeinen über 97 % reines m-Kresol. Der Siedepunkt von m-Kresol liegt bei 203°C, so dass entsprechend hohe Temperaturen für die Destillation des Reaktionsmediums gewählt werden müssen.

Im Hinblick auf den eingangs geschilderten Stand der Technik ist es beim erfindungsgemäßen Verfahren ausgesprochen überraschend, dass es mit den erfindungsgemäß einzusetzenden Basen als Katalysatoren und ohne organische Lösungsmittel gelingt, mit guten Ausbeuten aus 6-MSA selektiv die Carboxylgruppe abzuspalten und das bevorzugte Decarboxylierungsprodukt m-Kresol in hohen Ausbeuten und hohen Reinheiten zu erhalten.

Das bevorzugte Reaktionsprodukt m-Kresol ist ein wertvolles Zwischenprodukte zur Herstellung von Pflanzenschutzwirkstoffen und Pharmawirkstoffen. m-Kresol findet als Fungizid in der Landwirtschaft Anwendung. Kresole werden auch verwendet, um daraus Kunst- und Farbstoffe, Kunstharze (Kresolharze) und Arzneimittel herzustellen.

### Beispiele

### Allgemeine Arbeitsvorschrift:

1,0 g (6,6 mmol) 6-Methylsalicylsäure wurden mit der angegebenen Menge des zu verwendenden Katalysators innig vermengt und dann ohne Zugabe von Lösungsmittel unter inerter Atmosphäre auf 195 °C erhitzt. Das Gemisch wurde 6 h bei dieser Temperatur gehalten und dann auf Raumtemperatur abgekühlt. Das erhaltene Produktgemisch wurde mit 50 g Toluol extrahiert und das erhaltene Produktgemisch nach Silylierung mittels Gaschromatopgraphie untersucht und die Produktverhältnisse bestimmt.

In der Tabelle 1 bedeutet 5g Versuch, dass in obiger Versuchsvorschrift 5g 6-MSA mit 100 mol-% Katalysator umgesetzt wurden.

Für die Ermittlung der Ausbeute wurde die Vorschrift im fünffachen Maßstab durchgeführt und die erhaltene organische Produktphase vollständig vom Lösungsmittel befreit, um die Produktmenge per Auswaage zu bestimmen.

Das Produkt wurde dann nach Silylierung mittels Gaschromatographie untersucht. Die Ergebnisse der verschiedenen Umsätze sind in Tabelle 1 dargestellt. Jeweils angegeben ist der Umsatz von 6-MSA zu m-Kresol in Prozent.

**Tabelle 1**

| **Eingesetzte Menge des Katalysators** | | | | |
|---|---|---|---|---|
| **Katalysator** | 100 mol-% | 50 mol-% | 10 mol-% | 5g Versuch |
| **Basen** | | | | |
| Ca(OH)₂ | 100 | 100 | 100 | 34 |
| CaO | 100 | 100 | | 73 |
| Ba(OH)₂ | 100 | 100 | | 55 |
| Mg(OH)₂ | 100 | 100 | | 52 |
| KOH | 100 | 100 | 100 | 78 |

| **Sonstiges** | | | | |
|---|---|---|---|---|
| HCl (37%) - Stand der Technik | 41 | | | |
| Kein Katalysator | 34 | | | 12 |

100 mol-% bedeuten, dass relativ zu der eingesetzten Molmenge 6-Methylsalicylsäure die gleiche Molmenge (100 mol-%) des Katalysators eingesetzt wurden. 50 mol-% bedeuten, dass relativ zu der eingesetzten Molmenge 6-Methylsalicylsäure die halbe molare Menge (50 mol-%) des Katalysators eingesetzt wurden. 10 mol-% bedeuten, dass relativ zu der eingesetzten Molmenge 6-Methylsalicylsäure ein Zehntel der molaren Menge (10 mol-%) des Katalysators eingesetzt wurden.

Alle übrigen eingesetzten Base Katalysatoren sind für den Fachmann gängige und leicht zugängliche Edukte.

Die mit den erfindungsgemäßen Katalysatoren erzielten Ergebnisse zeigen überraschenderweise eine gegenüber dem Stand der Technik deutlich höhere Ausbeute an m-Kresol ohne den Eintrag von Schwermetallen.

## Patentansprüche

1. Verfahren zur Decarboxylierung von 6-MSA, **dadurch gekennzeichnet, dass** man als Katalysator wenigstens eine Base der Reihe Alkalimetallhydroxid, Erdalkalimetalloxid oder Erdalkalimetallhydroxid einsetzt.

2. Verfahren gemäß Anspruch 1, **dadurch gekennzeichnet, dass** man wenigstens eine Base der Reihe CaO, Ca(OH)₂, Ba(OH)₂, Mg(OH)₂, NaOH, LiOH oder KOH, einsetzt.

3. Verfahren gemäß der Ansprüche 1 oder 2, **dadurch gekennzeichnet, dass** man als Katalysator Ca(OH)₂ einsetzt.

4. Verfahren gemäß der Ansprüche 1 oder 2, **dadurch gekennzeichnet, dass** man als Katalysator Ba(OH)₂ einsetzt.

5. Verfahren gemäß der Ansprüche 1 oder 2, **dadurch gekennzeichnet, dass** man als Katalysator Mg(OH)₂ einsetzt.

6. Verfahren gemäß der Ansprüche 1 oder 2, **dadurch gekennzeichnet, dass** man als Katalysator KOH einsetzt.

7. Verfahren gemäß der Ansprüche 1 oder 2, **dadurch gekennzeichnet, dass** man als Katalysator CaO einsetzt.

8. Verfahren gemäß der Ansprüche 1 bis 7, **dadurch gekennzeichnet, dass** man dieses bei einer Temperatur im Bereich von 180°C-200°C, bevorzugt im Bereich von 190°C-200°C durchgeführt.

9. Verfahren gemäß der Ansprüche 1 bis 8, **dadurch gekennzeichnet, dass** man dieses in Gegenwart der 0,5- bis 15-fachen Gewichtsmenge Wasser, bezogen auf die einzusetzende Menge 6-MSA durchführt, bevorzugt beträgt diese Menge das 0,8- bis 6-fache.

10. Verfahren gemäß der Ansprüche 1 bis 9, **dadurch gekennzeichnet, dass** man dieses in Gegenwart zusätzlicher Metallkatalysatoren durchführt, bevorzugt in Gegenwart von Metallkatalysatoren auf Basis der Metalle der Reihe Zn, Fe, Cu, Ni, Co, Pd, Mo, Ru oder deren chemische Verbindungen mit Nichtmetallen bzw. Salze der Reihe Oxide, Chloride, Acetate oder deren Komplexe mit CO oder Dibenzylidenaceton.

11. Verfahren gemäß der Ansprüche 1 bis 10, **dadurch gekennzeichnet, dass** das Reaktionsprodukt m-Kresol ist.

12. Verfahren gemäß Anspruch 11, **dadurch gekennzeichnet, dass** man das nach der Decarboxylierung vorliegende Reaktionsmedium direkt destilliert und das m-Kresol isoliert.

13. Verwendung von wenigstens einer Base der Reihe Alkalimetallhydroxid, Erdalkalimetalloxid oder Erdalkalimetallhydroxid, bevorzugt CaO, Ca(OH)₂, Ba(OH)₂, Mg(OH)₂, KOH, LiOH oder NaOH, als Katalysator zur Decarboxylierung von 6-MSA, bevorzugt zur Herstellung von m-Kresol durch Decarboxylierung von 6-MSA.
